# EUROPÄISCHE PATENTANMELDUNG

(11) **EP 2 883 500 A1**
(43) Veröffentlichungstag der Anmeldung: **17.06.2015**
(21) Anmeldenummer: 13005767.2
(22) Anmeldetag: 11.12.2013
(51) Int. Cl.: A61B 6/04, A61N 5/10

(54) **Patientenbestrahlungseinrichtung**

(71) Anmelder: Buck Engineering & Consulting GmbH, 72764 Reutlingen (DE)
(72) Erfinder: Buck, Matthias, 72764 Reutlingen (DE)
(74) Vertreter: Reinhardt, Annette

(57) **Zusammenfassung**

Eine Patientenbestrahlungseinrichtung (1,31) besitzt eine Strahlenquelle (2, 32), aus der im Betrieb ein Strahl (4) an einer Austrittsöffnung (3) in einer Austrittsrichtung (22, 43, 44, 45, 46) austritt. Die Patientenbestrahlungseinrichtung (1, 31) besitzt eine Patientenpositioniereinrichtung mit einer Patientenaufnahme, die an einer Verfahranordnung im Raum bewegbar angeordnet ist. Die Verfahranordnung besitzt einen Befestigungsbereich (21), an dem die Verfahranordnung ortsfest im Raum gehalten ist. Die Patientenbestrahlungseinrichtung (1, 31) besitzt eine gedachte Ebene (23, 3 9, 40, 41, 42), die senkrecht zur Austrittsrichtung (22, 43, 44, 45, 46) liegt und die Austrittsöffnung (3) enthält. Um eine möglichst geringe Beaufschlagung der Patientenpositioniereinrichtung mit Strahlung zu erreichen, ist vorgesehen, dass der Befestigungsbereich (21) in mindestens einer im Betrieb vorgesehenen Stellung der Strahlenquelle (2, 32) mindestens teilweise auf der Seite der gedachten Ebene (23, 39, 40, 41, 42) liegt, die dem an der Austrittsöffnung (3) austretenden Strahl (4) abgewandt liegt.

## Beschreibung

Die Erfindung betrifft eine Patientenbestrahlungseinrichtung der im Oberbegriff des Anspruchs 1 angegebenen Gattung.

Aus der US 7,860,550 B2 ist eine Patientenbestrahlungseinrichtung bekannt, die eine Strahlenquelle und eine Patientenpositioniereinrichtung aufweist. Die Patientenbestrahlungseinrichtung kann ortsfest oder im Raum beweglich gehalten sein. Die Patientenpositioniereinrichtung ist so im Raum angeordnet, dass die Patientenpositioniereinrichtung und die Austrittsöffnung des Strahls auf gegenüberliegenden Seiten des Behandlungsbereichs oder bezogen auf den Behandlungsbereich winklig zueinander angeordnet sind.

Im Betrieb streut der von der Strahlenquelle ausgesandte Strahl. Dadurch wird auch die Patientenpositioniereinrichtung mit der Strahlung beaufschlagt. Dies kann die Lebensdauer der Patientenpositioniereinrichtung verringern.

Der Erfindung liegt die Aufgabe zugrunde, eine Patientenbestrahlungseinrichtung der gattungsgemäßen Art zu schaffen, die einen einfachen Aufbau und eine hohe Lebensdauer besitzt.

Diese Aufgabe wird durch eine Patientenbestrahlungseinrichtung mit den Merkmalen des Anspruchs 1 gelöst.

Es ist vorgesehen, dass der Befestigungsbereich der Patientenpositioniereinrichtung in mindestens einer im Betrieb vorgesehenen Stellung der Strahlenquelle mindestens teilweise auf der Seite einer gedachten Ebene liegt, die dem an der Austrittsöffnung austretenden Strahl abgewandt liegt. Dadurch wird die Strahlenbelastung der Patientenpositioniereinrichtung deutlich verringert. Die gedachte Ebene liegt dabei senkrecht zur Austrittsrichtung des Strahls und enthält die Austrittsöffnung. Es hat sich überraschend gezeigt, dass durch die Positionierung des Befestigungsbereichs mindestens teilweise auf der dem austretenden Strahl abgewandt liegenden Seite der gedachten Ebene die Reichweite der Patientenpositioniereinrichtung gegenüber der Austrittsöffnung nicht verringert ist. Vielmehr kann eine Patientenpositioniereinrichtung mit geringerer Reichweite ausreichend sein, da für viele Anwendungsfälle eine Bestrahlung des zu bestrahlenden Gewebes nah an der Austrittsöffnung vorteilhaft ist. Durch die vorgesehene Anordnung des Befestigungsbereichs ergibt sich eine bessere Zugänglichkeit des Behandlungsbereichs für den Patienten und für medizinisches Personal, da die Patientenpositioniereinrichtung und die Strahlenquelle an der gleichen Seite des Raums angeordnet sind. Der Bereich des Raums, in den der Strahl gelenkt wird, kann dadurch frei zugänglich gestaltet werden. Der Befestigungsbereich kann dabei an der Decke, an der Wand oder am Boden angeordnet sein. Besonders bevorzugt ist eine Deckenmontage des Befestigungsbereichs, da dadurch der Boden frei zugänglich bleibt.

Vorteilhaft liegt der Befestigungsbereich in mindestens einer im Betrieb vorgesehenen Stellung der Strahlenquelle vollständig auf der dem an der Austrittsöffnung austretenden Strahl abgewandten Seite der gedachten Ebene. Dadurch wird eine besonders geringe Strahlenbelastung eines großen Bereichs der Patientenpositioniereinrichtung erreicht.

Ein einfacher Aufbau der Patientenbestrahlungseinrichtung kann erreicht werden, wenn die Verfahranordnung einen Roboterarm umfasst, der mindestens fünf Rotationsbewegungsachsen besitzt. Besonders vorteilhaft weist der Roboterarm mindestens sechs Rotationsbewegungsachsen auf. Der Roboterarm kann dabei ein Standardroboterarm sein, wie er in Industrieanwendungen üblicherweise zum Einsatz kommt. Die Verfahranordnung kann jedoch zusätzlich oder alternativ eine oder mehrere Linearbewegungsachsen aufweisen, die Translationsbewegungen in eine oder mehrere Richtungen erlauben.

Für einen Roboterarm ist vorzugsweise vorgesehen, dass der Roboterarm ein Grundgestell besitzt, das den Befestigungsbereich aufweist. Der Befestigungsbereich ist dabei der Bereich, mit dem der Roboterarm im Raum, beispielsweise an einer Raumdecke oder am Boden, fixiert ist. Der Befestigungsbereich kann beispielsweise Öffnungen zur Aufnahme von Befestigungsschrauben oder dgl. aufweisen.

An dem Grundgestell ist vorteilhaft ein Karussell um eine erste Rotationsbewegungsachse schwenkbar gelagert. Eine vorteilhafte Anordnung ergibt sich, wenn die erste Rotationsbewegungsachse in mindestens einer im Betrieb vorgesehenen Stellung der Strahlenquelle und in mindestens einer, vorzugsweise in jeder Stellung des Roboterarms auf der dem Strahl abgewandten Seite der gedachten Ebene angeordnet ist. Vorzugsweise sind auch die Lager, mit denen das Karussell am Grundgestell um die erste Rotationsbewegungsachse schwenkbar gelagert ist, auf der dem Strahl abgewandten Seite der gedachten Ebene angeordnet. Vorzugsweise ist das Karussell in mindestens einer im Betrieb vorgesehenen Stellung der Strahlenquelle und in mindestens einer, vorteilhaft in jeder Stellung des Roboterarms vollständig auf der dem Strahl abgewandten Seite der gedachten Ebene angeordnet.

Vorteilhaft ist an dem Karussell eine Schwinge um eine zweite Rotationsbewegungsachse schwenkbar gelagert. Die zweite Rotationsbewegungsachse ist dabei insbesondere senkrecht zur ersten Rotationsbewegungsachse ausgerichtet. Auch eine Schrägstellung der ersten zur zweiten Rotationsbewegungsachse, also ein Winkel zwischen den beiden Achsen, der weniger als 90° beträgt, kann jedoch vorteilhaft sein. Auch die Schwinge ist vorteilhaft in mindestens einer im Betrieb vorgesehenen Stellung der Strahlenquelle und in mindestens einer, vorzugsweise in jeder Stellung des Roboterarms mindestens teilweise auf der dem Strahl abgewandten Seite der gedachten Ebene angeordnet.

An der Schwinge ist vorteilhaft ein Arm um eine dritte Rotationsbewegungsachse schwenkbar gelagert. An dem Arm ist vorteilhaft ein Handabschnitt angeordnet, an dem die Patientenaufnahme gehalten ist. Der Handabschnitt ermöglicht vorteilhaft eine Bewegung der Patientenaufnahme gegenüber dem Arm um drei Rotationsbewegungsachsen. Der Aufbau der Patientenpositioniereinrichtung mit einem Roboterarm mit einem Grundgestell, einem Karussell, einer Schwinge, einem Arm und einem Handabschnitt ermöglicht, dass Teile der Patientenpositioniereinrichtung, nämlich vorzugsweise das Grundgestell und das Karussell sowie die Schwinge mindestens teilweise auf der dem Strahl abgewandten Seite der gedachten Ebene der Patientenbestrahlungseinrichtung angeordnet sind. Lediglich der Arm und der Handabschnitt werden im Betrieb meist so anzuordnen sein, dass sie sich auf der Seite der gedachten Ebene befinden, auf der der Strahl sich befindet, um eine Positionierung des Patienten im gewünschten Bereich zu ermöglichen.

Die Patientenaufnahme ist vorzugsweise eine Patientenliege. Es kann jedoch auch eine andere Art der Patientenaufnahme, beispielsweise ein Stuhl oder dgl., vorgesehen sein. Bevorzugt ist die Strahlenquelle ortsfest im Raum angeordnet. Dadurch kann der Befestigungsbereich so angeordnet werden, dass er immer mindestens teilweise auf der dem Strahl abgewandt liegenden Seite der gedachten Ebene angeordnet ist. Der Strahl kann dabei vorzugsweise horizontal, vertikal von oben oder von unten oder in einem festen Winkel zur Horizontalen, beispielsweise in einem Winkel von 45°, ausgerichtet sein.

Es kann jedoch auch vorgesehen sein, dass die Strahlenquelle beweglich im Raum angeordnet ist. Die Austrittsöffnung ist dabei vorzugsweise auf einer kreisbogenförmigen Bahn im Raum beweglich. Der Befestigungsbereich befindet sich dabei vorteilhaft mindestens teilweise auf der dem Strahl abgewandten Seite der gedachten Ebene, wenn sich die Austrittsöffnung in einem Kreissegment befindet, das sich über einen Winkel von mindestens 90° um den Kreismittelpunkt des Kreisbogens erstreckt. Dadurch kann die Strahlenbelastung des Roboterarms gegenüber bekannten Anordnungen, bei denen sich der Befestigungsbereich immer auf der dem Strahl zugewandten Seite der gedachten Ebene befindet, deutlich verringert werden.

Ausführungsbeispiele der Erfindung werden im Folgenden anhand der Zeichnung erläutert. Es zeigen:
- Fig. 1: eine perspektivische Darstellung einer Patientenbestrahlungseinrichtung,
- Fig. 2 bis Fig. 5: Ansichten der Patientenbestrahlungseinrichtung etwa von oben in unterschiedlichen Positionen der Patientenliege,
- Fig. 6 bis Fig. 10: perspektivische Darstellungen der Patientenbestrahlungseinrichtung etwa in der in den Figuren 1 und 2 gezeigten Ausrichtung der Patientenaufnahme,
- Fig. 11: eine schematische Seitenansicht eines Ausführungsbeispiels der Patientenbestrahlungseinrichtung.

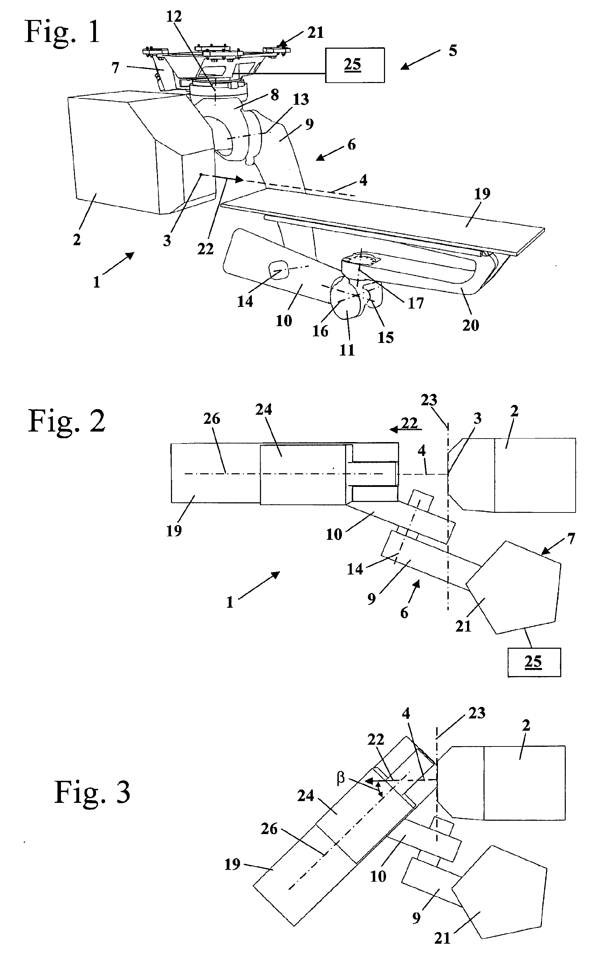

Fig. 1 zeigt schematisch eine Patientenbestrahlungseinrichtung 1. Die Patientenbestrahlungseinrichtung 1 umfasst eine Strahlenquelle 2, die beispielsweise eine Strahlenquelle für Photonen, Protonen, Neutronen oder Ionen sein kann. Die Patientenbestrahlungseinrichtung 1 kann zur Diagnose oder zur Therapie vorgesehen sein. Die Strahlenquelle 2 ist in den Figuren lediglich schematisch dargestellt und kann beispielsweise ein Zyklotron oder Synchrotron zur Strahlerzeugung umfassen. Die Strahlenquelle 2 besitzt eine Austrittsöffnung 3, aus der ein Strahl 4 in einer Austrittsrichtung 22 austritt. Der Strahl 4 kann dabei streuen, wobei die Austrittsrichtung 22 die mittlere Richtung des Strahls 4 bezeichnet. Zur Positionierung eines Patienten im Strahl 4 ist eine Patientenpositioniereinrichtung 5 vorgesehen. Die Patientenpositioniereinrichtung 5 umfasst im Ausführungsbeispiel einen Roboterarm 6. Anstatt des Roboterarms kann auch eine andere Patientenpositioniereinrichtung, beispielweise ein an Linearachsen verfahrbarer Tisch oder dgl., vorgesehen sein. Der Roboterarm 6 besitzt ein Grundgestell 7, das einen Befestigungsbereich 21 besitzt, mit dem der Roboterarm 6 an einer nicht gezeigten Raumdecke festgelegt ist. Der Roboterarm 6 kann jedoch auch am Boden oder an einer Wand im Raum festgelegt sein.

An dem Grundgestell 7 ist ein Karussell 8 um eine erste Rotationsbewegungsachse 12 schwenkbar gelagert. Im Ausführungsbeispiel ist die erste Rotationsbewegungsachse 12 senkrecht ausgerichtet. Am Karussell 8 ist eine Schwinge 9 um eine zweite Rotationsbewegungsachse 13 schwenkbar gelagert. Die zweite Rotationsbewegungsachse 13 ist senkrecht zur ersten Rotationsbewegungsachse 12 ausgerichtet. Im Ausführungsbeispiel verläuft die zweite Rotationsbewegungsachse 13 horizontal. An der Schwinge 9 ist ein Arm 10 um eine dritte Rotationsbewegungsachse 14 schwenkbar gelagert. Die dritte Rotationsbewegungsachse 14 ist parallel zur zweiten Rotationsbewegungsachse 13 ausgerichtet. Am Arm 10 ist ein Handabschnitt 11 angeordnet. Am Handabschnitt 11 ist ein Bügel 20 einer Patientenliege 19 festgelegt. Der Handabschnitt 11 ist als Gelenk ausgebildet und erlaubt eine Verschwenkung des Bügels 20 mit der Patientenliege 19 um eine vierte Rotationsbewegungsachse 15, eine fünfte Rotationsbewegungsachse 16 und eine sechste Rotationsbewegungsachse 17. Dadurch kann die Patientenliege 19 so verschwenkt werden, dass sie horizontal oder in einem gewünschten Winkel im Raum liegt. Auch eine andere Anordnung der Rotationsbewegungsachsen 12 bis 17 kann jedoch vorteilhaft sein. Die Patientenbestrahlungseinrichtung 1 besitzt eine Steuereinrichtung 25 zur Ansteuerung der Patientenpositioniereinrichtung 5. Die Steuereinrichtung 25 ist in Fig. 1 lediglich schematisch gezeigt und kann an geeigneter Stelle positioniert werden.

Wie Fig. 2 zeigt, besitzt die Patientenbestrahlungseinrichtung 1 eine gedachte Ebene 23. Die gedachte Ebene 23 enthält die Austrittsöffnung 3 des Strahls 4 und ist senkrecht zur Austrittsrichtung 22 ausgerichtet. Wie Fig. 2 zeigt, ist der Roboterarm 6 im Raum neben der Strahlenquelle 2 positioniert. Der Befestigungsbereich 21 befindet sich vollständig auf der dem Strahl 4 abgewandten Seite der gedachten Ebene 23. Dadurch wird eine geringe Belastung des Befestigungsbereichs 21 und des gesamten Roboterarms 6 mit Streustrahlung des austretenden Strahls 4 erreicht. Bei der in Fig. 2 gezeigten Anordnung der Patientenliege 19 sind auch das Grundgestell 7 und das Karussell 8 vollständig auf der dem Strahl 4 abgewandten Seite der gedachten Ebene 23 angeordnet. In Fig. 2 ist das Karussell 8 vom Grundgestell 7 verdeckt. Lediglich ein Teil der Schwinge 9 sowie der Arm 10 und der Handabschnitt 11 mit der Patientenliege 19 und dem Bügel 20 sind auf der Seite der gedachten Ebene 23 angeordnet, auf der sich der Strahl 4 befindet. In der in Fig. 2 gezeigten Position der Patientenliege 19 ist eine Längsachse 26 der Patientenliege 19 parallel zum Strahl 4, also parallel zur Austrittsrichtung 22 angeordnet. Ein in Fig. 2 schematisch gezeigter Patient 24 ist dadurch ebenfalls in Längsrichtung des Strahls 4 ausgerichtet. Dadurch kann beispielsweise eine Bestrahlung des Patienten 24 von oben durch die Schädeldecke erfolgen.

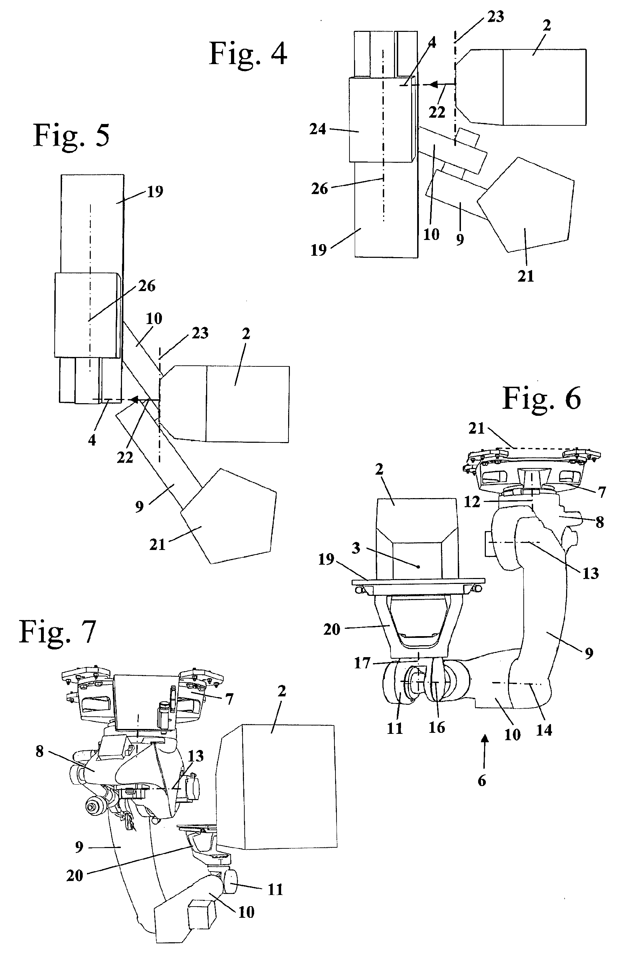

In Fig. 3 ist die Patientenliege 19 gegenüber der in Fig. 2 gezeigten Stellung so verschwenkt, dass die Längsachse 26 mit der Austrittsrichtung 22 einen Winkel β einschließt, der beispielsweise etwa 30° bis etwa 60° betragen kann. Fig. 4 zeigt die Patientenliege 19 in einer Ausrichtung, in der die Längsachse 26 senkrecht zur Austrittsrichtung 22 steht. Bei der in Fig. 5 gezeigten Anordnung steht die Längsachse 26 ebenfalls senkrecht zur Austrittsrichtung 22, allerdings wird der Patient 26 von der anderen Seite bestrahlt. Wie die Figuren 2 bis 5 zeigen, ist bei der gezeigten Anordnung des Befestigungsbereichs 21 an der dem Strahl 4 abgewandten Seite der gedachten Ebene 23 eine Bestrahlung des Patienten 24 aus allen horizontalen Richtungen möglich. Bei Schrägstellung der Patientenliege 19 ist auch eine Bestrahlung aus schrägen Richtungen möglich. Trotz der Anordnung des Befestigungsbereichs 21 auf der dem Strahl 4 abgewandten Seite der gedachten Ebene 23, also auf der Seite, die von der Austrittsöffnung 3 gesehen in Gegenrichtung zur Austrittsrichtung 22 liegt, ergibt sich eine große Bewegungsfreiheit bei der Positionierung der Patientenliege 19.

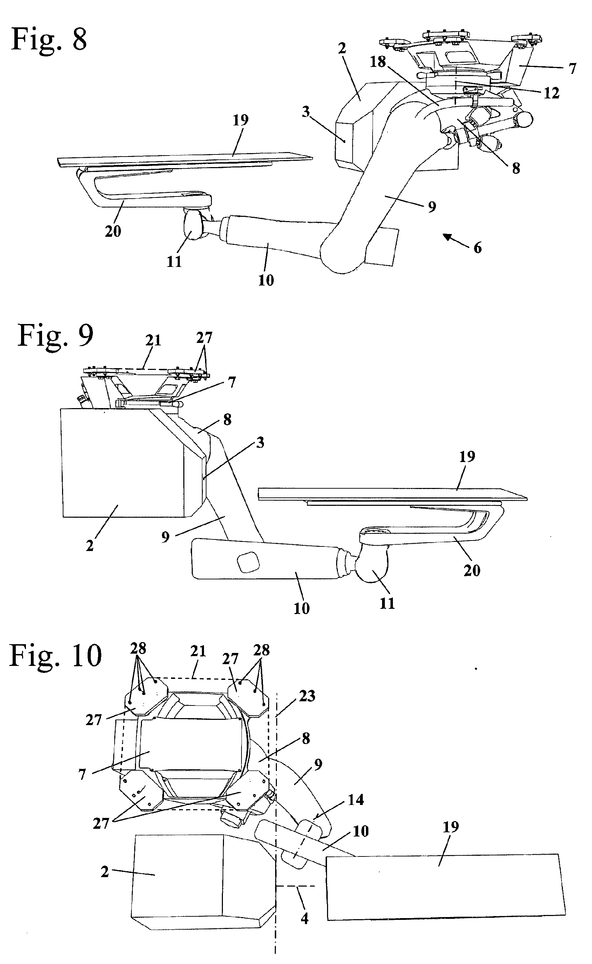

Die Figuren 6 und 7 zeigen die Ausrichtung der Rotationsbewegungsachsen 12 bis 14, 16 und 17. In Fig. 6 ist auch die Austrittsöffnung 3 des Strahls 4 gezeigt. Wie Fig. 8 zeigt, weist der Roboterarm 6 eine Leitungsführung 18 auf, die als Kabelkanal ausgebildet sein kann und durch die Versorgungsleitungen zum Antrieb des Roboterarms 6, Versorgungsleitungen zur Versorgung des Patienten, beispielsweise mit Anästhesiegas oder dgl., sowie Sensor- und Aktorleitungen geführt sein können.

Die Figuren 9 und 10 zeigen die Anordnung des Befestigungsbereichs 21 auf der dem Strahl 4 abgewandten Seite der gedachten Ebene 23. Wie Fig. 10 zeigt, wird der Befestigungsbereich 21 durch vier Befestigungsplatten 27 gebildet, die jeweils mehrere Schrauböffnungen 28 zur Aufnahme von Befestigungsschrauben besitzen. Im Ausführungsbeispiel ist der Befestigungsbereich 21 schematisch als der die Befestigungsplatten 27 einschließende Bereich eingezeichnet. Der Befestigungsbereich 21 liegt dann mindestens teilweise auf der dem Strahl 4 abgewandten Seite der gedachten Ebene 23, wenn mindestens ein Abschnitt mindestens einer der Befestigungsplatten 27 auf der dem Strahl 4 abgewandten Seite der gedachten Ebene 23 liegt. Im Ausführungsbeispiel sind alle vier Befestigungsplatten 27 auf der dem Strahl 4 abgewandten Seite der gedachten Ebene 23 angeordnet. Auch das Grundgestell 7 und das Karussell 8 sind vollständig an der dem Strahl 4 abgewandten Seite der gedachten Ebene 23 angeordnet. Die Schwinge 9 ist teilweise auf dieser Seite der gedachten Ebene 23 angeordnet und wird von der gedachten Ebene 23 geschnitten.

Im Ausführungsbeispiel nach den Figuren 1 bis 10 ist die Strahlenquelle 2 ortsfest im Raum angeordnet. Der Befestigungsbereich 21 ist so angeordnet, dass der Befestigungsbereich 21 vollständig auf der dem Strahl 4 abgewandten Seite der gedachten Ebene 23 liegt. Dadurch wird eine vergleichsweise geringe Beaufschlagung des Roboterarms 6 mit Streustrahlung aus der Strahlenquelle 2 erreicht. Lediglich die nah an der Patientenliege 19 angeordneten Bereiche des Roboterarms 6, nämlich der Handabschnitt 11, der Arm 10 und ein Abschnitt der Schwinge 9 können teilweise von gestreuten Partikeln des Strahls 4 getroffen werden. Dadurch, dass die Strahlenquelle 2 und der Befestigungsbereich 21 mit dem Grundgestell 7 auf der gleichen Seite der gedachten Ebene 23 angeordnet sind, wird eine sehr geringe Beaufschlagung dieses Bereichs des Roboterarms 6 mit Streustrahlung des Strahls 4 erreicht.

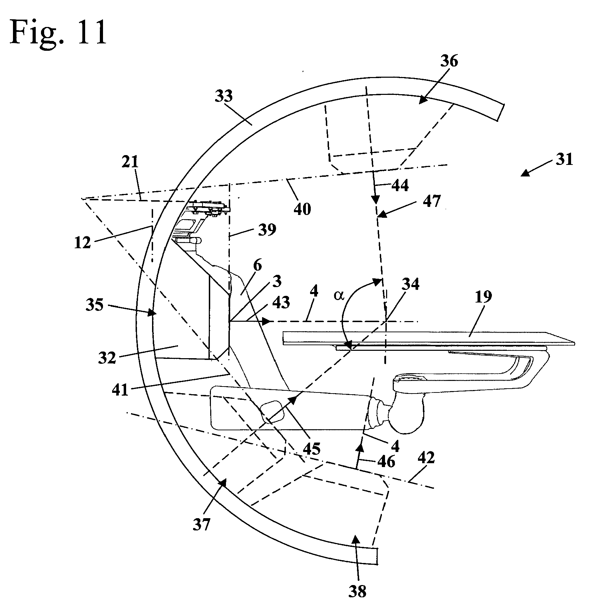

Fig. 11 zeigt ein Ausführungsbeispiel einer Patientenbestrahlungseinrichtung 31 mit einer Strahlenquelle 32. Die Strahlenquelle 32 kann wie die Strahlenquelle 2 eine Strahlenquelle für Photonen wie Röntgenstrahlung, für Protonen oder für Ionen sein. Die Strahlenquelle 32 ist im Raum beweglich angeordnet. Die Patientenbestrahlungseinrichtung 31 besitzt eine Schiene 33, an der die Strahlenquelle 32 verfahrbar ist. Der Roboterarm 6 ist am Befestigungsbereich 21 ortsfest im Raum angeordnet. Mit durchgezogener Linie ist in Fig. 11 eine erste Stellung 35 der Strahlenquelle 32 eingezeichnet. Der Strahl 4 tritt in dieser ersten Stellung 35 horizontal in einer Austrittsrichtung 43 aus der Austrittsöffnung 3 aus. Die Patientenbestrahlungseinrichtung 31 besitzt eine gedachte Ebene 39, die senkrecht zur Austrittsrichtung 43 in der ersten Stellung 35 verläuft und die die Austrittsöffnung 3 der Strahlenquelle 32 in der ersten Stellung 35 enthält. Der Befestigungsbereich 21 des Roboterarms 6 ist vollständig auf der dem Strahl 4 abgewandten Seite der gedachten Ebene 39 angeordnet.

Die Schiene 33 bildet einen Teilkreisbogen um einen Kreismittelpunkt 34. Beim Verfahren der Strahlenquelle 32 an der Schiene 33 bewegt sich die gedachte Ebene mit. Der Strahl 4 geht in jeder Stellung der Strahlenquelle 32 durch den Kreismittelpunkt 34. In Fig. 11 ist mit gestrichelter Linie eine zweite Stellung 36 der Strahlenquelle 32 eingezeichnet. In der zweiten Stellung 36 tritt der Strahl 4 in einer Austrittsrichtung 44 aus der Strahlenquelle 32 aus. In der zweiten Stellung 36 besitzt die Patientenbestrahlungseinrichtung 31 eine gedachte Ebene 40, die senkrecht zur Austrittsrichtung 44 angeordnet ist und die Austrittsöffnung 3 in der zweiten Stellung 36 de Strahlenquelle 32 enthält. Die gedachte Ebene 40 schneidet in der zweiten Stellung 36 der Strahlenquelle 32 den Befestigungsbereich 21. Ein sehr geringer Abschnitt des Befestigungsbereichs 21 ist in der zweiten Stellung 36 der Strahlenquelle 32 auf der dem Strahl 4 abgewandten Seite der gedachten Ebene 40 angeordnet. Bei der zweiten Stellung 36 ist der Strahl 4 nach unten ausgerichtet, gegenüber der Senkrechten jedoch geringfügig geneigt. Wird die Strahlenquelle 32 weiter von der ersten Stellung 35 weg verfahren, so bewegt sich die gedachte Ebene weiter, und der Befestigungsbereich 21 gelangt vollständig auf die Seite der gedachten Ebene, auf der auch der Strahl 4 angeordnet ist.

Fig. 11 zeigt eine dritte Stellung 37 der Strahlenquelle 32, bei der der Strahl 4 schräg nach oben ausgerichtet ist. In der dritten Stellung 37 tritt der Strahl 4 in einer Austrittsrichtung 45 aus der Strahlenquelle 32 aus. Die senkrecht zur Austrittsrichtung 45 ausgerichtete, gedachte Ebene 41 schneidet den Befestigungsbereich 21. Ein geringer Abschnitt des Befestigungsbereichs 21 liegt auf der dem Strahl 4 abgewandten Seite der gedachten Ebene 41. Wird die Strahlenquelle 32 weiter an der Schiene 33 verfahren, so bewegt sich auch die gedachte Ebene. In einer in Fig. 11 schematisch gezeigten vierten Stellung 38 ist der Strahl 4 steil nach oben geneigt. Der Strahl 4 tritt in dieser Stellung 38 in einer Austrittsrichtung 46 aus der Austrittsöffnung 3 aus. Die gedachte Ebene 42, die die Austrittsöffnung 3 in dieser Stellung enthält und senkrecht zur Austrittsrichtung 46 angeordnet ist, schneidet den Befestigungsbereich 21 nicht mehr. In der vierten Stellung 38 ist der Befestigungsbereich 21 vollständig auf der dem Strahl 4 zugewandten Seite der gedachten Ebene 42 angeordnet. Die Stellungen 36 und 37 bezeichnen Stellungen, in denen ein geringer Abschnitt des Befestigungsbereichs 21 noch an der dem Strahl 4 abgewandten Seite der gedachten Ebene 40 bzw. 41 angeordnet ist. Der Strahl 4 schließt zwischen der Ausrichtung in der zweiten Stellung 36 und der Ausrichtung in der dritten Stellung 37 einen Winkel α ein, der vorzugsweise mehr als 90° beträgt. Der Winkel α beträgt insbesondere mehr als etwa 120°. Solange sich die Austrittsöffnung 3 in einem Kreissegment 47 befindet, das sich von der Position der Austrittsöffnung 3 in der zweiten Stellung 36 bis zur Position der Austrittsöffnung 3 in der dritten Stellung 37 erstreckt, liegt mindestens ein Abschnitt des Befestigungsbereichs auf der dem Strahl 4 abgewandten Seite der gedachten Ebene 39, 40, 41. Dadurch wird auch bei einer sich bewegenden Strahlenquelle 32 eine vergleichsweise geringe Strahlenbelastung des Roboterarms 6 erreicht.

Die vorgeschlagene Anordnung des Befestigungsbereichs 21 ist auch für ortsfeste Strahlenquellen zweckmäßig, bei denen der Strahl 4 nicht horizontal, sondern senkrecht ausgerichtet oder in einem Winkel gegenüber der Horizontalen geneigt ist.

## Patentansprüche

1. Patientenbestrahlungseinrichtung mit einer Strahlenquelle (2, 32), an der im Betrieb ein Strahl (4) an einer Austrittsöffnung (3) in einer Austrittsrichtung (22, 43, 44, 45, 46) austritt, und mit einer Patientenpositioniereinrichtung (5), wobei die Patientenpositioniereinrichtung (5) eine Patientenaufnahme aufweist, die an einer Verfahranordnung im Raum bewegbar angeordnet ist, wobei die Verfahranordnung einen Befestigungsbereich (21) besitzt, an dem die Verfahranordnung ortsfest im Raum gehalten ist, wobei die Patientenbestrahlungseinrichtung (1, 31) eine gedachte Ebene (23, 39, 40, 41, 42) besitzt, die senkrecht zur Austrittsrichtung (22, 43, 44, 45, 46) liegt und die Austrittsöffnung (3) enthält,
**dadurch gekennzeichnet, dass** der Befestigungsbereich (21) in mindestens einer im Betrieb vorgesehenen Stellung (35, 36, 37) der Strahlenquelle (2, 32) mindestens teilweise auf der Seite der gedachten Ebene (23, 39, 40, 41) liegt, die dem an der Austrittsöffnung (3) austretenden Strahl (4) abgewandt liegt.

2. Patientenbestrahlungseinrichtung nach Anspruch 1,
**dadurch gekennzeichnet, dass** der Befestigungsbereich (21) in mindestens einer im Betrieb vorgesehenen Stellung (35) der Strahlenquelle (2, 32) vollständig auf der dem an der Austrittsöffnung (3) austretenden Strahl (4) abgewandten Seite der gedachten Ebene (23, 39) liegt.

3. Patientenbestrahlungseinrichtung nach Anspruch 1 oder 2,
**dadurch gekennzeichnet, dass** die Verfahranordnung einen Roboterarm (6) umfasst, der mindestens fünf Rotationsbewegungsachsen (12, 13, 14, 15, 16, 17) besitzt.

4. Patientenbestrahlungseinrichtung nach Anspruch 3,
**dadurch gekennzeichnet, dass** der Roboterarm (6) ein Grundgestell (7) besitzt, das den Befestigungsbereich (21) aufweist.

5. Patientenbestrahlungseinrichtung nach Anspruch 4,
**dadurch gekennzeichnet, dass** an dem Grundgestell (7) ein Karussell (8) um eine erste Rotationsbewegungsachse (12) schwenkbar gelagert ist.

6. Patientenbestrahlungseinrichtung nach Anspruch 5,
**dadurch gekennzeichnet, dass** die erste Rotationsbewegungsachse (12) in mindestens einer im Betrieb vorgesehenen Stellung (35) der Strahlenquelle (2) auf der dem Strahl (4) abgewandten Seite der gedachten Ebene (23, 39) angeordnet ist.

7. Patientenbestrahlungseinrichtung nach Anspruch 5 oder 6,
**dadurch gekennzeichnet, dass** das Karussell (8) an mindestens einer im Betrieb vorgesehenen Stellung (35) der Strahlenquelle (2, 32) und mindestens einer Stellung des Roboterarms (6) vollständig auf der dem Strahl (4) abgewandten Seite der gedachten Ebene (23, 39) angeordnet ist.

8. Patientenbestrahlungseinrichtung nach einem der Ansprüche 5 bis 7,
**dadurch gekennzeichnet, dass** an dem Karussell (8) eine Schwinge (9) um eine zweite Rotationsbewegungsachse (13) schwenkbar gelagert ist, wobei die zweite Rotationsbewegungsachse (13) senkrecht zur ersten Rotationsbewegungsachse (12) ausgerichtet ist.

9. Patientenbestrahlungseinrichtung nach Anspruch 8,
**dadurch gekennzeichnet, dass** die Schwinge (9) in mindestens einer im Betrieb vorgesehenen Stellung (35) der Strahlenquelle (2, 32) und in mindestens einer Stellung des Roboterarms (6) mindestens teilweise auf der dem Strahl (4) abgewandten Seite der gedachten Ebene (23, 39) angeordnet ist.

10. Patientenbestrahlungseinrichtung nach Anspruch 8 oder 9,
**dadurch gekennzeichnet, dass** an der Schwinge (9) ein Arm (10) um eine dritte Rotationsbewegungsachse (14) schwenkbar gelagert ist, und dass an dem Arm (10) ein Handabschnitt (11) angeordnet ist, an dem die Patientenaufnahme gehalten ist, wobei der Handabschnitt (11) eine Bewegung der Patientenaufnahme gegenüber dem Arm (10) um drei Rotationsbewegungsachsen (15, 16, 17) ermöglicht.

11. Patientenbestrahlungseinrichtung nach einem der Ansprüche 1 bis 10,
**dadurch gekennzeichnet, dass** die Patientenaufnahme eine Patientenliege (19) ist.

12. Patientenbestrahlungseinrichtung nach einem der Ansprüche 1 bis 11,
**dadurch gekennzeichnet, dass** die Strahlenquelle (2) ortsfest im Raum angeordnet ist.

13. Patientenbestrahlungseinrichtung nach einem der Ansprüche 1 bis 11,
**dadurch gekennzeichnet, dass** die Strahlenquelle (32) beweglich im Raum angeordnet ist.

14. Patientenbestrahlungseinrichtung nach Anspruch 13,
**dadurch gekennzeichnet, dass** die Austrittsöffnung (3) auf einer kreisbogenförmigen Bahn im Raum beweglich ist, und dass der Befestigungsbereich (21) sich mindestens teilweise auf der dem Strahl (4) abgewandten Seite der gedachten Ebene (39, 40, 41) befindet, wenn sich die Austrittsöffnung (3) in einem Kreissegment (47) befindet, das sich über einen Winkel (α) von mindestens 90° um den Kreismittelpunkt (34) des Kreisbogens erstreckt.
